# EUROPEAN PATENT APPLICATION

(11) **EP 1 050 339 A1**
(43) Date of publication of application: **08.11.2000**
(21) Application number: 99201421.7
(22) Date of filing: 07.05.1999
(51) Int. Cl.: B01J 23/58, B01J 37/02, C07C 45/00

(54) **Stable catalysts and processes for making and using the same**

(71) Applicant: DSM N.V., 6411 TE Heerlen (NL)
(72) Inventor: Milone, Candida, 98155 Messina (IT); Lefferts, Leonardus, 6191 ES Beek (NL)

(57) **Abstract**

The present invention relates to reactively stable catalysts, to the preparation thereof, and to reaction processes using these catalysts. These stable catalysts are useful in hydrogenation reactions, in particular, hydrogenation of phenols to yield the corresponding cyclohexanone. A process for forming a supported palladium catalyst wherein said support material is metal-doped by impregnating the support material with a group IA or IIA metal bicarbonate solution; drying the bicarbonate impregnated support material and calcining the bicarbonate-treated support material to form a group IA or IIA metal-doped support material.

## Description

### FIELD OF THE INVENTION

The present invention relates to reactively stable catalysts, to the preparation thereof, and to reaction processes using these catalysts. More specifically, the present invention relates to stable catalysts useful in hydrogenation reactions, in particular, hydrogenation of phenols to yield the corresponding cyclohexanone.

### BACKGROUND OF THE INVENTION

Important aspects of stable catalysts include its activity lifetime and its selectivity to the desired product. A short activity lifetime, i.e., high deactivation rate, means high frequency of interruption of the process to regenerate or replace the catalyst which, of course, increases operating times and costs. Transition metal catalysts, which are in widespread use by the petroleum and petrochemical industry, are particularly prone to deterioration during prolonged operation. Many attempts have been made to overcome this problem by trying to extend the activity lifetime of such catalysts.

For example, palladium-containing supported catalysts and their use in the hydrogenation of phenol to cyclohexanone are described in German Published Specification No. 2,045,882, and British Patent Specification No. 1,368,419 wherein the preparation of which aluminum spinel supporting materials, such as lithium, magnesium, cobalt, manganese or zinc spinels, which have been calcined at a high temperature are employed. In such catalysts, the catalytically active substances are generally distributed irregularly over the whole supporting material. As a consequence of this, when these catalysts are employed industrially, undesirable differences in activity, selectivity of conversion and service life can occur. Furthermore, it frequently happens that substances present near to the center of a support particle have either no influence at all or only a minor influence on the reaction to be catalyzed. In the case of valuable catalytically active substances, for example in the case of noble metals and compounds thereof, this results in unnecessarily high catalyst costs.

European Published Specification No. 0012,153 and U.S. Patents 4,407,733 and 4,774,221 describe, *inter alia,* supported catalysts in which an inert supporting material having a BET surface area less than 20 m²/g is impregnated to saturation with a solution of a base and dried to constant weight, then impregnated with a metal salt solution, also until saturation is reached, and finally reduced to the metal. The base being applied in an amount such that, before impregnation with the metal salt solution, 0.01 to 50 gram equivalents of base per gram equivalent of metal are present in the supporting material. Catalysts prepared by this process contain the catalytically active substances within a narrow, annular zone in the interior of these small surface area inert supporting materials and also have a relatively short activity lifetime.

German Published Specification No. 2,752,291 also describes the hydrogenation of phenol to give cyclohexanone. In this case catalysts consisting of carbon or charcoal particles coated with palladium and having an (inner) surface area of 100 to 2,000 m²/g, and a phenol containing a small amount of alkali compounds are employed. Based on the interaction of the promoter contained in the phenol with the catalyst, the effect achieved is, for example, that, after a hydrogenation time of 210 minutes, a product containing 1.1% by weight of cyclohexanol, 87.5% by weight of cyclohexanone and 11.4% by weight of phenol is obtained, whereas, if the promoter in the feed phenol is omitted under otherwise identical conditions, a product containing 1.4% by weight of cyclohexanol and only 65.9% by weight of cyclohexanone with 32.5% by weight of phenol remaining is obtained (see Example 1 in German Published Specification No. 2,752,291).

Finally, hydrogenations of phenol are also described in U.S. Pat. No. 3,076,810. It is proved by means of examples that, using a phenol containing 0.01 part of sodium carbonate in 1,000 parts and using a catalyst containing 5% of palladium on charcoal, as well as 5,000 ppm of sodium, a product mixture having the composition 97.2% of cyclohexanone, less than 0.5% of phenol and remainder cyclohexanol is obtained (see Example 1A). Varying the amount of sodium in the catalyst alters the cyclohexanol content (maximum approximately 3%) in the product only to an immaterial extent (see Examples 1B and 1D).
Products having a high content of cyclohexanol (maximum 80%) are only obtained if a palladium/charcoal catalyst doped with sodium and phenol containing a relatively large proportion of sodium carbonate are employed (see Example 4).
This state of the art also leads away from the present invention, since it suggests that the feed phenol, and not the catalyst, should be varied in order to increase the selectivity and conversion of the catalyst.

However, all these processes and the catalysts obtainable thereby still suffer from considerable disadvantages. These catalysts have support materials that are understood to interfere with the catalyst activity by forming by-products that prematurely coke up the catalytically active metal and/or these catalysts are disadvantaged by the arrangement of the catalytically active metal which is exposed to abrasion losses and poisoning. These factors seem to contribute to the high deactivation rate of these relatively unstable catalysts.

### SUMMARY OF THE INVENTION

In accordance with the present invention, there is provided a palladium catalyst comprising a group IA or IIA metal-doped support material which has long, stable activity lifetime. A process for forming a supported palladium catalyst wherein said support material is metal-doped by impregnating the support material with at least one group IA or IIA metal bicarbonate in a solution; drying the bicarbonate impregnated support material and calcining the bicarbonate-treated support material to form a group IA or IIA metal-doped support material. A further embodiment of the present invention provides a supported palladium catalyst as discussed above wherein the group IA or IIA metal-doped support material is palladium treated by further impregnating the support material with a palladium salt solution, drying and calcining. The invention also provides very diverse catalytic processes which include, for example, hydrogenation, dehydrogenation, hydrogenolysis, oxidation, oligomerization, polymerization, isomerisation, dehydrogenation and cyclization utilizing this catalyst.

### BRIEF DESCRIPTION OF THE DRAWINGS

Figure 1 is a graph reporting the catalytic activity of exemplary and comparative catalysts tested under integral conditions.

Figure 2 is a graph reporting the catalytic activity of exemplary and comparative catalysts tested under integral conditions.

Figure 3 is a graph reporting the catalytic activity of comparative catalysts tested under integral conditions.

### DETAILED DESCRIPTION OF THE INVENTION

Materials useful as support materials for the supported catalysts of the present invention include, for example, metal oxides, silicates, spinels, carbides, carbonates or mixtures thereof. Preferred supporting materials are aluminum oxides, silicon dioxides, silicon dioxide/aluminum oxide mixtures, amorphous silicas, kieselguhrs, barium, strontium or calcium carbonates, mixtures thereof optionally containing, in addition, silicon dioxides or aluminum oxides, titanium oxides, zirconium oxides, magnesium oxides, magnesium silicates, zirconium silicates, magnesium/aluminum spinels, silicon carbides, tungsten carbides, mixtures of silicon carbides with silicon dioxides, or any desired mixtures of the above mentioned materials. Aluminum oxide is particularly preferred. The supporting materials can be used in a very wide variety of forms, for example as spheres, granules, extrudates, tablets, saddle-shaped bodies, tubular sections, fragments and/or honeycomb ceramics. Although support materials of widely varying shapes and sizes may be useful, typically, the nominal size of the support material particles is between 0.1 mm to 40 mm, preferably 1 mm to 10 mm. The inert supporting material preferably has a BET surface area of between 20 and 300 m²/g, more preferably between 50 and 200 m²/g, and most preferably between 70 and 160 m²/g.

In the pre-treatment stage, the inert support material is metal-doped with a group IA or IIA metal bicarbonate solution by contacting the support material with the bicarbonate solution in any number of a variety of ways. "Group IA or IIA" herein refers to the metals within the respective group numbers of the Periodic Table of the Elements (CRC Handbook of Chemistry and Physics, 78^{th} ed. 1997-1998). The following group IA or IIA metals may be mentioned individually by way of example: Li, Na, K, Rb, Cs, Be, Mg, Ca, Sr, and Ba. Of these, Na, K and Cs are preferred, with Na being particularly preferred. Although the group IA or IIA metal is discussed herein in the singular preferred form, it will be appreciated that the support can be doped with one or more group IA or IIA metals either individually in sequence or as mixture of two or more simultaneously.

In general, the doping of the support material may be carried out at temperatures of 5° to 100°C, preferably at 10° to 60°C and particularly preferentially at 20° to 40°C. The treatment can be carried out at normal pressure, under elevated pressures or under reduced pressure. The ratio of the group IA or IIA metal to the palladium, also referred to herein as "the metal", is 0.01 to 50, preferably 0.5 to 20 gram equivalents and particularly preferentially 0.5 to 10 gram equivalents (based on the palladium desired in the final product). The bicarbonate solution and the support material should remain in contact for at least 5 minutes but typically not longer than 72 hours, preferably between about 2 to 12 hours. The preferred procedure for treating the support material with a group IA or IIA metal bicarbonate solution is known as incipient wetness impregnation wherein the support is saturated with a bicarbonate solution in, for example, a rotavapor at a reduced pressure until evaporation of the solvent is substantially complete.

The group IA or IIA metal bicarbonate can be brought to act in very diverse forms on the inert support materials. The group IA or IIA metal bicarbonate can be employed either solvent-free in the liquid form or in the form of solutions in aqueous or non-aqueous solvents. It can sometimes be appropriate for the solvent used to form a homogeneous phase with the metal bicarbonate, but this is in no way necessary for carrying out the process according to the invention. Solvents which can be useful include, for example: water, straight-chain or branched hydrocarbons with 1 to 12 and preferably with 1 to 6 carbon atoms which contain a hydroxyl group, keto group and/or carboxyl group, cyclic hydrocarbons with 5 to 7 and preferably 6 carbon atoms in the ring system which contains a hydroxyl group, keto group and/or carboxyl group and heterocyclic compounds with preferably 6 atoms in the ring system which contain a hydroxyl group, keto group and/or carboxyl group and contain oxygen and/or nitrogen as hetero-atoms.

Particularly preferred solvents include water, alcohols, such as methanol, ethanol, propanol, isopropanol, butanol, ethylene glycol and glycerol, and also ketones, such as acetone, and mixtures thereof, hydrochloric acid, sulfuric acid, nitric acid, hydriodic acid, hydrobromic acid, acetic acid and formic acid.

The bicarbonate solution can be added, or metered, into the inert support, or vice versa. Preferably, the base solution is metered into the support.

After the pretreatment of the inert support materials with the group IA or IIA metal bicarbonate solution, the treated support is subsequently dried in a manner which is in itself known. For example, the support may be dried discontinuously or continuously in a drying cabinet or in a stream of warm air at temperatures of about 50° to 200°C, and preferably at 100° to 150°C. Temperatures of 105° to 125°C and pressures of 1 atm are particularly preferred. All pressures indicated herein are absolute pressures. The support material loaded with group IA or IIA metal bicarbonate are dried in this way to a residual moisture content of less than 10%, preferably less than 5%, particularly preferentially less than 2% and very particularly preferentially less than 1% of the absorbency of the support.

After the group IA or IIA metal-doped support is dried, it is calcined by exposure to an environment having a temperature greater than 200°C and below the melting point of the support, preferably greater than 250°C and less than 700°C, for a period of time sufficient to achieve at least partial thermal decomposition of the metal-doped support. Typically, the calcination step is conducted in air for a period of 1 to 12 hours, preferably 2 to 6 hours. The calcination step may be performed as a step separate from the drying step or as a continuation of the drying step by raising the drying temperature suitably and maintaining the metal-doped support in this environment for a period of time sufficient to achieve at least partial calcination.

In a subsequent process stage (or palladium treatment stage), the support material is treated with a palladium salt solution by solution-support contact in a manner similar to that described above. In general, the metal-doped support material may be treated by the palladium solution at temperatures of 5° to 100°C, preferably at 10° to 60°C and particularly preferentially at 20° to 40°C. The treatment can be carried out at normal pressure, under elevated pressures or under reduced pressure. The palladium salt solution and the support material should typically remain in contact for at least 5 minutes but typically not longer than 72 hours, preferably between about 2 to 12 hours. The preferred procedure for treating the metal-doped support material with palladium is known as incipient wetness impregnation wherein the support is saturated with a palladium salt solution in, for example, a rotavapor at a reduced pressure until evaporation of the solvent is substantially complete.

More particularly, the group IA or IIA metal-doped support material is impregnated, in accordance with its absorbency, to saturation with a palladium salt solution under conditions which, although independent, fall within the parameters discussed hereinabove except with a palladium salt solution in place of the bicarbonate solution. The palladium metal concentration of the solution(s) employed in this way are calculated so that the desired metal concentrations on the support materials are obtained either by a single impregnation or by multiple impregnations. The calculated palladium concentration is used to determine the requisite group IA or IIA metal to be employed for the pretreatment of the support.

The palladium can be employed in the form of a salt or complex salt, either as a solution or as a suspension. Examples of salts and/or complexes of palladium which may be useful include: Na₂[PdCl]₄, PdCl₂, PdBr₂, PdI₂, H₂PdCl₄, K₂[PdCl₆], Pd(CH₃COO)₂, Pd(acac)₃, Pd(NO₃)₂, PdSO₄ and [Pd(NH₃)₂]Cl₂, with Pd(acac)₃, PdCl₂, Pd(NO₃)₂ or Na₂[PdCl]₄ being preferred, and Pd(acac)₃, or PdCl₂ being particularly preferred. A halogen-free catalyst is another preferred embodiment of the present invention and is achieved by using one of the non-halogen containing palladium salts such as Pd(acac)₃, or Pd(NO₃)₂ as the palladium precursor. The solutions or suspensions may include one or more of the above noted aqueous or non-aqueous solvents noted above, with water being the preferred solvent for the palladium salt solution.

The chemical compositions of the palladium salt solutions are in-part determined by the nature of the catalysts to be prepared. The solutions may contain in addition to palladium one or more additional metals including other catalytically active metals, dissolved in the form of their salts. Physical parameters, such as, for example, the absorbency of the support and the solubility of the palladium salt solution can make it necessary to carry out repeated solution-support contact steps, for instance impregnations, in order to obtain the required concentration of active substance in the finished catalyst. For example, the concentration of the palladium salt solution should be adjusted so that the finished catalyst contains 0.1 to 20 wt.%, preferably 0.3 to 5 wt.%, and more preferably 0.5 to 2 wt.% of palladium (based on the metal in its elemental form) , a side from any other additional active metals that optionally may be present.

After treating the metal-doped support material with palladium, the support material can be dried and calcined in accordance with the conditions herein discussed with respect to the drying and calcining steps in the pre-treatment stage. Although the conditions and preferred ranges discussed herein are applicable to the respective steps for the palladium treatment stage, it should be appreciated that each of these steps are independent and as such may employ the same or, typically, different conditions than those employed in the corresponding step.

Once the palladium containing, group IA or IIA metal-doped support material is obtained it is still typically desired to have the palladium reduced to the metal. This reduction can be carried out at any time after the support is treated with the palladium salt solution. Preferably, this reduction is carried out immediately after the calcination step or *in situ,* that is to say immediately before carrying out the reaction to be catalyzed by the catalyst. This reduction can be carried out in any conventional manner and using reducing agents which are customary for reductions of this type. Examples of suitable reducing agents include hydrazine hydrate, hydrogen, formaldehyde and sodium borohydride. Preferred reducing agents are gaseous hydrogen and aqueous hydrazine hydrate.

In addition to the steps mentioned above, a wide variety of optional steps may be introduced providing they do not adversely effect the advantages provided by the present invention. These additional steps include calcining the support material by itself prior to the above process and/or grinding the support material either before or after pre-treatment or before or after palladium treatment. Other steps may include washing or rinsing the support materials either before or after the drying and/or calcining steps in either the pretreatment or palladium treatment stages or even after reduction of the palladium. Preferably, the washing is continued until the wash water is substantially free of ions from the sodium bicarbonate and/or anions of the palladium salt.

The possibility of carrying out the process according to the invention industrially is illustrated with the aid of the preparation of a palladium-containing Al₂O₃ catalyst.

A quantity of Al₂O₃ particles is pretreated, in accordance with its absorbency, with a solution of a bicarbonate solution, for example sodium bicarbonate in H₂O, in a ratio of 0.01 to 50 equivalents of sodium per equivalent of palladium to be added (prior to the actual impregnation with the solution of palladium salt). The support material is then dried at a temperature of 100° to 200°C, for example 120° C, to a residual moisture content of < 10%, for example < 1%, of the absorbency of the support. Thereafter, the sodium-doped support is calcined in air at a temperature greater than 300°C but lower than 700°C, for a period of between 2 to 4 hours. After completing the pre-treatment stage, the group IA or IIA metal-doped support particles are impregnated, in accordance with its absorbency (by processes which are in themselves known) with a solution of a palladium salt, for example palladium acetate in toluene, the metal content of the solution being adjusted to correspond to the amount previously considered to determine the concentration of the bicarbonate solution. After a reaction time of from a few hours to several days, for example 24 hours, the palladium containing, sodium-doped support material is dried and calcined. If necessary, the metal salt applied is first reduced to the metal by known methods, for example by treatment with H₂ flow at between 100° to 400°C for 1-6 hours.

Although not wishing to be held to any particular theory, the reactive stability enjoyed by the catalysts of the present invention may be understood by noting the relatively low metal surface area of less than 0.9 m²/g, preferably 0.7 m²/g or less, and the relatively high mean particle size of between 60 to 120 Å, preferably more than about 70 Å. When used industrially, it is believed that the catalysts obtained in this way will experience minimal poisoning and minimal loss of palladium activity. As a result of this, it is expected that a very long catalyst life with constant activity is obtained.

The supported catalysts prepared according to the invention can be used for very diverse catalytic processes, such as, for example, hydrogenation, dehydrogenation, hydrogenolysis, oxidation, oligomerization, polymerization, isomerisation or cyclization. In these catalytic reactions, the supported catalysts prepared by the process according to the invention can be employed either in the sump phase, the trickle phase or the gas phase. The trickle phase and the gas phase are preferred, with gas phase being particularly preferred. The reactions can be carried out either under normal pressure or excess pressure or under reduced pressure. Catalytic hydrogenation reactions are a preferred field of use for the supported catalysts according to the invention. They are particularly suitable for hydrogenating aromatic systems in the nucleus and for hydrogenating certain substituents, for example nitro groups on aromatic systems. The supported catalysts according to the invention are preferably used in the catalytic hydrogenation of phenol and substituted phenols, it being possible to hydrogenate the aromatic system and/or the substituent. The use, which is particularly preferred, of supported catalysts according to the invention is the hydrogenation of phenols to yield the corresponding cyclohexanones.

The catalyzed reactions according to the invention can be carried out under customary reaction conditions and in customary reactors. For example, the hydrogenation according to the invention can be carried out with readily vaporizable phenols in the gas phase and in tubular reactors, and with phenols which are difficult to vaporize in the down-flow liquid phase and in vertical reactors. The reaction temperatures can be, for example, between 100° and 350°C. They are preferably between 120° and 250°C. The pressure during the reaction can be, for example, between 1 and 350 atm. When the reaction is carried out in the gas phase, relatively low pressures, for example pressures within the range of from 1 to 5 atm, are generally preferred. When the reaction is carried out in the down-flow liquid phase, relatively high pressures, for example pressures within the range from 100 to 350 atm, are generally preferred.

In general, at least 2 moles of hydrogen are fed to the reactor per mole of the particular phenol employed. It is preferable to introduce into the reactor 4 or 12 moles of hydrogen per mole of the particular phenol employed. When the hydrogenation according to the invention is carried out in the gas phase, the procedure followed is, for example, to vaporize the phenol in the feed stream of hydrogen and then to pass this combined stream into the reactor. If catalysts in which it is still necessary to reduce the palladium compounds present before the admission of a phenol are employed in the hydrogenation process according to the invention, this reduction can be carried out in a particularly simple manner by first allowing only hydrogen to flow over the catalyst located in the reactor and not introducing the particular phenol until the palladium compounds have been reduced.

For example, the active substance combination of palladium on a group IA or IIA metal-doped Al₂O₃ support, which has been treated by the process according to the invention, finds preferred application in the catalytic hydrogenation of aromatic compounds, such as, for example, phenol or substituted phenol compounds to the corresponding cyclohexanone or substituted cyclohexanone compounds. This hydrogenation reaction regardless of the which phase is employed is typically conducted by passing the compound to be reduced over a fixed catalyst. The reaction is advantageously carried out with an excess of hydrogen. Examples of phenols which can be employed in the hydrogenation reaction according to the invention include those represented by formula (I), as follows: 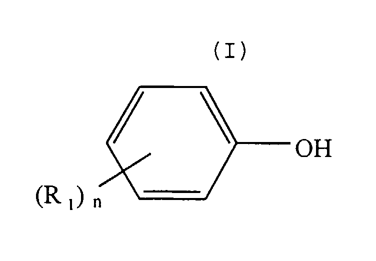 in which
R₁ independently represents halo, nitro, an unsubstituted or amino substituted C₁ to C₆ alkyl or C₆ to C₁₀ aryl, optionally substituted amino, C₁ to C₆ alkoxy or C₆ to C₁₀ aroxy, and
n represents zero or an integer from 1 to 5, but, in the event that R₁ = nitro, n only represents zero, 1 or 2.

Halo here includes fluorine, chlorine, bromine or iodine atoms. Optionally substituted amino may include, for example, an NH₂ group which is optionally monosubstituted or disubstituted by C₁ to C₆ alkyl or C₆ to C₁₀ aryl.

R₁ preferably represents methyl, ethyl, n-propyl, i-propyl, n-hexyl, phenyl, hydroxyl, chlorine, bromine, NH₂, methoxy, ethoxy or phenoxy and n preferably represents zero, 1 or 2. It is particularly preferable for R₁ to represent methyl in the meta-position and/or para-position and for n to represent zero or 1.

It is also possible, for example, to employ phenols represented by formula (II), as follows: 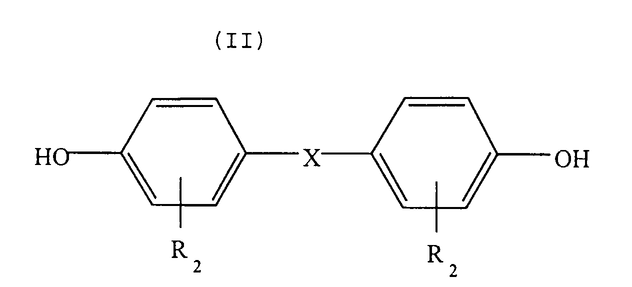 in which
the R₂'s independently of one another each represent a hydrogen atom, C₁ to C₆ alkyl, C₁ to C₆ alkoxy or halogen, and
X represents -CH₂-, -C(CH₃)₂-, -cyclohexyl-, -CO-, -S-, -SO₂-, -O- or a single bond.

Preferably, the R₂'s independently represent a hydrogen atom or methyl and X represents -C(CH₃)₂-.

The use of phenols of the formula (I) is preferred to the use of phenols of the formula (II). The hydrogenation process according to the invention is preferably carried out using catalysts which can be obtained by the measures described hereinabove.

The products leaving the reactor are generally cooled, and the hydrogenated phenols are separated off in a liquid or solid form. They can, if appropriate, be purified further by customary methods, for example by distillation. The excess of hydrogen which is generally present can be recycled to the reactor, it being preferable to discharge a small proportion.

The hydrogenation, according to the invention, of phenols by means of the supported catalysts according to the invention surprisingly affords products containing, very preponderantly, in general to the extent of over 90% by weight and frequently to the extent of over 95% by weight, the corresponding cyclohexanones, and only very little, in general less than 8%, of the corresponding cyclohexanols. The formation of other by-products being very slight; the service lives of the catalysts are long and stable.

Other reaction wherein the palladium catalysts of the present invention may be useful include the production of alpha-picoline and 2,3-lutidine prepared in accordance with the following reaction schemes: 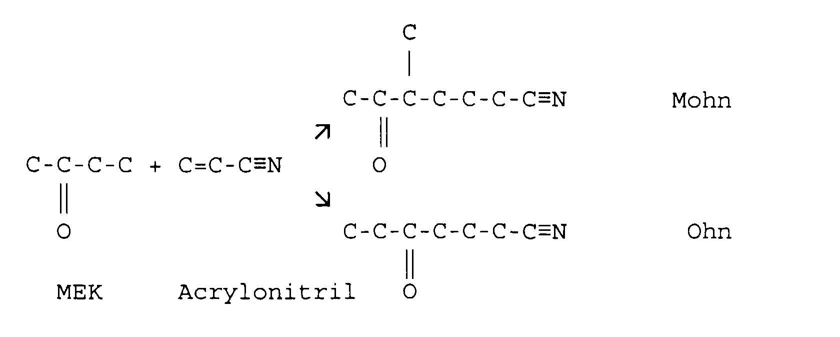 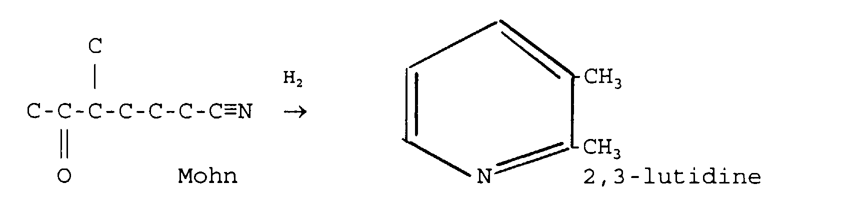 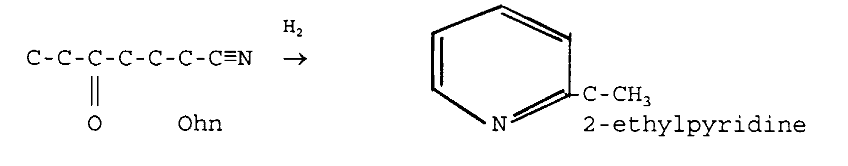 Reactiecondities alpha pic, 2,3 lutidine
Temperatuur: 240-260°C
molverhouding H₂/docan of H₂/Mohn = 4-5
Totaal druk 1-1.6 atm
LHSV 0.15s⁻¹

The present invention will be hereinafter described in more detail by way of examples which are given for illustration of the present invention and shall not be construed as limiting the present invention.

### EXAMPLES 1-2 AND COMPARATIVE EXAMPLES 1-9

A series of aluminum oxide (Al₂O₃) supports, commercially available from Engelhard as AlTO104 having an 1/8" nominal particle and a 90 m²/g surface, was ground to a powder having an average particle size in the range of 0.180-0.125 mm and pretreated with three different types of sodium precursors. The supports were sodium-doped by incipient wetness impregnation in a rotavapor at 30°C. After impregnation, all samples were dried at 120°C for 3 hours and then calcined at 400°C for 4 hours. The type of sodium precursor used and amounts are presented in Table 1.

In the palladium treatment stage, the sodium doped supports were treated by incipient wetness impregnation with either a toluene solution of Pd acetate or an aqueous solution of palladium chloride to achieve a palladium content of approximately 1 wt.%, relative to the total weight of the catalyst inclusive of the support. After impregnation with the palladium solution, the catalyst were oven dried at 120°C overnight and then calcined in air at 400°C for 2 hours. Finally, the catalysts were reduced under hydrogen flow at 200°C for 2 hours.

The resulting catalyst were analyzed and compared with comparative 1, a conventional palladium-containing hydrogenation catalyst (commercially available from Johnson-Matthey), for palladium surface area, by CO chemisorption using a pulse technique at room temperature, determined by assuming a stoichiometry of Pd/CO=1 and a Pd surface density of 1.27 x 10¹⁹ atom/m²(wherein the average metal particles size was calculated by the equation d=6V/S where S= palladium metal surface area and V=volume of palladium bulk. The catalyst properties are summarized in Table 1.

**TABLE 1**

| | Palladium Precursor | Sodium Precursor | Palladium (wt.%) | Sodium (wt.%) | Metal Surface Area (m²/g) | Mean Particle Diameter (A) |
|---|---|---|---|---|---|---|
| Ex. 1 | Pd (acac)₃ | NaHCO₃ | 1 | 2 | 0.7 | 71 |
| Ex. 2 | PdCl₂ | NaHCO₃ | 1 | 2 | 0.5 | 99 |
| Comp. 1 | PdCl₂ | Na acetate | 1 | 1 | 0.9 | 55 |
| Comp. 2 | Pd (acac)₃ | NaHCO₃ | 1 | 0.5 | 1.8 | 28 |
| Comp. 3 | Pd (acac)₃ | NaHCO₃ | 1 | 1 | 1.1 | 45 |
| Comp. 4 | PdCl₂ | NaNO₃ | 1 | 2 | 0.5 | 99 |
| Comp. 5 | PdCl₂ | NaNO₃ | 1 | 1 | 0.9 | 55 |
| Comp. 6 | PdCl₂ | Na₃PO₄ | 1 | 0.5 | 1.3 | 38 |
| Comp. 7 | PdCl₂ | Na₃PO₄ | 1 | 1 | 0.9 | 55 |
| Comp. 8 | PdCl₂ | Na₃PO₄ | 1 | 2 | 0.9 | 55 |

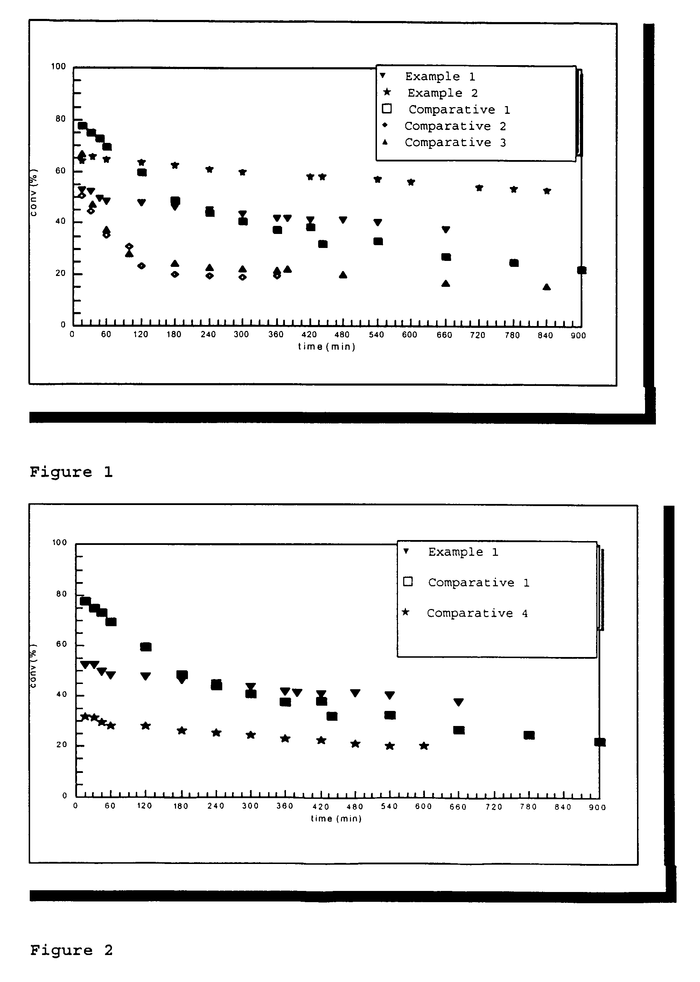

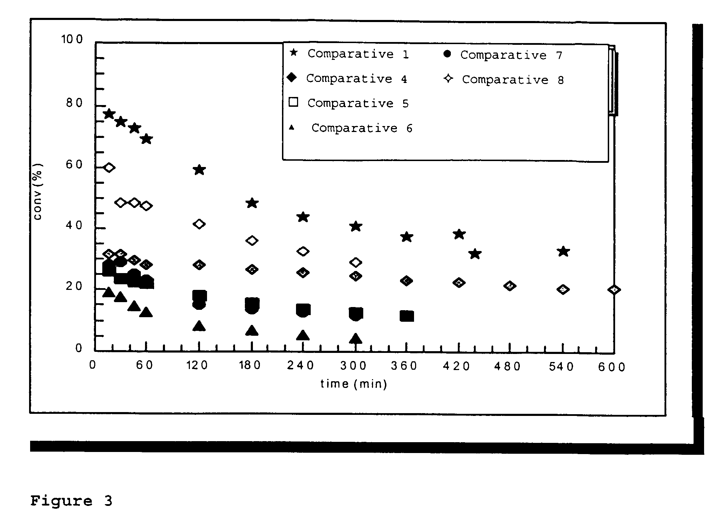

The catalytic activity of these catalysts were tested under integral conditions in order to determine the deactivation rates of these catalysts. The partial pressure of phenol and H₂ were maintained at 0.76 atm. and 0.39 atms, respectively. The cyclohexanone partial pressure was 0.018 atm. The reaction temperature was 160°C and the amount of catalyst was 0.420 g. Therefore, the Weight High Space Velocity (WHSV) was 9 gₚₕₑₙₒₗ/g_{catalyst}-h. The results of these tests are reported in Figures 1, 2 and 3.

Having described specific embodiments of the present invention, it will be understood that many modifications thereof will readily appear or may be suggested to those skilled in the art, and it is intended therefore that this invention is limited only by the spirit and scope of the following claims.

## Claims

1. A catalyst comprising:
a palladium containing, group IA or IIA metal-doped support material wherein said support material is metal-doped by:
i) impregnating said support material with a group IA or IIA metal bicarbonate solution;
ii) drying said bicarbonate impregnated support material to constant weight; and
iii) calcining said bicarbonate-treated support material to form a group IA or IIA metal-doped support material.

2. The catalyst according to claim 1, wherein said group IA or IIA metal-doped support material is palladium treated by:
i) further impregnating said group IA or IIA metal-doped support material with a palladium salt solution;
ii) further drying said palladium salt impregnated support material to constant weight; and
iii) further calcining said palladium treated support material.

3. The catalyst according to claim 1, wherein said group IA or IIA metal bicarbonate includes sodium bicarbonate, potassium bicarbonate or cesium bicarbonate.

4. The catalyst according to claim 1, wherein said group IA or IIA metal bicarbonate includes sodium bicarbonate.

5. The catalyst according to claim 1, wherein said palladium salt solution includes Pd(acac)₃, PdCl₂, Pd(NO₃)₂ or Na₂[PdCl]₄.

6. The catalyst according to claim 1, wherein said palladium salt solution includes Pd(acac)₃.

7. The catalyst according to claim 1, wherein said catalyst is halogen-free.

8. The catalyst according to claim 1, wherein said group IA or IIA metal is present in an amount greater than 1 wt.%.

9. The catalyst according to claim 1, wherein said group IA or IIA metal is present in an amount greater than 1.5 wt.%.

10. The catalyst according to claim 1, wherein said group IA or IIA metal is present in an amount greater than 2 wt.%.

11. The catalyst according to claim 1, wherein palladium is present in an amount between 0.5 and 2 wt.%.

12. The catalyst according to claim 1, wherein said catalyst has a mean particle diameter of greater than 50 Å.

13. The catalyst according to claim 1, wherein said catalyst has a mean particle diameter of greater than 60 Å.

14. The catalyst according to claim 1, wherein said catalyst has a palladium metal surface area of less than 1.0 m²/g.

15. The catalyst according to claim 1, wherein said catalyst has a palladium metal surface area of between 0.5 and 1.0 m²/g.

16. The catalyst according to claim 1, wherein said palladium is present as a metal.

17. A hydrogenation catalyst prepared by:
(a) group IA or IIA metal-doping an inert support material, by:
i) impregnating said support material with a group IA or IIA metal bicarbonate solution;
ii) drying said metal-treated support material to constant weight; and
iii) calcining said metal-treated support material to form a group IA or IIA metal-doped support material; and
(b) treating said metal-doped support material with a palladium salt solution, by:
i) further impregnating said support material with a palladium salt solution;
ii) further drying said palladium salt impregnated support material to constant weight; and
iii) further calcining said palladium-treated support material.

18. A process for preparing a hydrogenation catalyst comprising:
a) impregnating a support material with a group IA or IIA metal bicarbonate solution;
b) drying said sodium impregnated support material to constant weight;
c) calcining said sodium treated support material to form a group IA or IIA metal-doped support material;
d) further impregnating said metal-doped support material with a palladium salt solution;
e) further drying said palladium salt impregnated support material to constant weight; and
f) further calcining said palladium-treated support material.

19. The process of claim 18, wherein said metal-doped support material is impregnated with said palladium salt solution until saturation is reached.

20. The process of claim 18, further comprising washing said palladium-treated support material with water.

21. The process of claim 20, wherein said washing is continued until the wash water is substantially free of ions from the sodium bicarbonate and anions of the palladium salt.

22. The process of claim 18, further comprising reducing said palladium present on said palladium-treated support material to palladium metal.

23. The process of claim 22, wherein said reduction is carried out with hydrazine hydrate, hydrogen, formaldehyde or sodium borohydride.

24. The process of claim 18, wherein said support material is selected from aluminum oxides, silicon dioxides, silicon dioxide/aluminum oxide mixtures, amorphous silicas, kieselguhrs, mixtures thereof optionally containing, in addition, silicon dioxides or aluminum oxides, titanium oxides, zirconium oxides, magnesium oxides, magnesium silicates, zirconium silicates, magnesium/aluminum spinels, silicon carbides, tungsten carbides, mixtures of silicon carbides with silicon dioxides or any desired mixtures of the abovementioned materials.

25. The process of claim 1, wherein the support material is Al₂O₃.

26. The process of claim 18, wherein said hydrogenating catalyst comprises 0.01 to 50_gram equivalents of a group IA or IIA metal per gram equivalent of palladium (reduced).

27. A process for forming cyclohexanone comprising: exposing a catalyst in accordance with claim 1 to phenol in the presence of hydrogen.
